# EUROPEAN PATENT APPLICATION

(11) **EP 3 171 210 A1**
(43) Date of publication of application: **24.05.2017**
(21) Application number: 15822027.7
(22) Date of filing: 01.07.2015
(51) Int. Cl.: G02B 7/08, A61B 1/00, G02B 7/04

(54) **LENS-DRIVING APPARATUS**

(30) Priority: 16.07.2014 JP 2014146196
(71) Applicant: OLYMPUS CORPORATION, Hachioji-shi Tokyo 192-8507 (JP)
(72) Inventor: FUJISAWA Yutaka, Hachioji-shi, Tokyo 192-8507 (JP); UNSAI Hiroshi, Hachioji-shi, Tokyo 192-8507 (JP); NISHIHARA Teruyuki, Hachioji-shi, Tokyo 192-8507 (JP); SUGA Takeshi, Hachioji-shi, Tokyo 192-8507 (JP)
(74) Representative: Schicker, Silvia
(86) International application number: PCT/JP2015/069002
(87) International publication number: WO 2016/009842

(57) **Abstract**

A lens driving apparatus includes: a lens 16a; a soft iron 31 that holds the lens 16a to be movable between an end part and the other end part; magnets 33 and 34 that attract and hold the soft iron 31 when the soft iron 31 comes into contact with one of the end part and the other end part; a driver circuit 22 that generates a drive signal bringing the soft iron 31 into contact with one of the end part and the other end part; an operation section 11 that instructs the driver circuit 22 to move the soft iron 31 to bring the soft iron into contact with one of the end part and the other end part; and a timing generation circuit 21 that generates a drive signal causing the soft iron 31 to move in an instructed direction and supplies the drive signal to the driver circuit 22, and controls the driver circuit 22 to periodically generate the drive signal causing the soft iron 31 to move toward an instructed end part even after the soft iron 31 comes into contact with the instructed end part.

## Description

### Technical Field

The present invention relates to a lens driving apparatus, and in particular to a lens driving apparatus that periodically generates a drive signal causing a lens holding section to move.

### Background Art

An endoscope system that includes an endoscope, processor, and the like has been conventionally widely used in a medical field, an industrial field, and other fields. The endoscope picks up an image of an object inside a subject, and the processor generates an observation image of the object picked up by the endoscope.

An endoscope that includes, for example, an electromagnetic actuator at a distal end, drives a focus lens and a zoom lens with use of the electromagnetic actuator, and accordingly performs focus adjustment and zoom observation, has been used.

For example, Japanese Patent Application Laid-Open Publication No. 2009-204714 discloses an image pickup apparatus that causes a lens holder housing a lens, to move between a normal position and a macro position, thereby being capable of performing focus adjustment.

For example, when the lens holder is displaced from the normal position to the macro position, the image pickup apparatus first applies a long pulse signal requiring a long application time period, to a coil once, and subsequently applies a short pulse signal requiring a short application time period, to the coil multiple times, thereby causing the lens holder to move toward the macro position. Thereafter, when the lens holder has moved to the macro position, the image pickup apparatus stops application of the pulse.

In the above-described image pickup apparatus, however, when large impact is applied to the electromagnetic actuator while the pulse signal is not applied, the electromagnetic actuator may move unintentionally. In this case, disadvantageously, the above-described image pickup apparatus may not maintain the intended focus state and the intended observation field, and may be put into unintended endoscope observation state.

Therefore, an object of the present invention is to provide a lens driving apparatus that allows for stable endoscope observation even if any impact is applied to an actuator.

### Disclosure of Invention

### Means for Solving the Problem

A lens driving apparatus according to an aspect of the present invention includes: a first lens; a first lens holding section that holds the first lens to be movable between an end part and another end part; a first magnet that is provided on the end part and the other end part, and attracts and holds the first lens holding section when the first lens holding section comes into contact with one of the end part and the other end part; a first drive signal generation section that generates a drive signal bringing the first lens holding section into contact with one of the end part and the other end part; an instruction section that instructs the first drive signal generation section to move the first lens holding section to bring the first lens holding section into contact with the end part or move the first lens holding section to bring the first lens holding section into contact with the other end part; and a drive signal control section that generates a drive signal causing the first lens holding section to move in a direction instructed by the instruction section and supplies the drive signal to the first drive signal generation section, and controls the first drive signal generation section to periodically generate the drive signal causing the first lens holding section to move toward the instructed end part even after the first lens holding section comes into contact with the instructed end part.

### Brief Description of the Drawings

Fig. 1 is a diagram illustrating a configuration of an endoscope apparatus configuring a lens driving apparatus according to a first embodiment;
Fig. 2 is a cross-sectional diagram to explain configurations of an electromagnetic actuator 15 and a focus lens unit 16;
Fig. 3 is a diagram to explain an example of a driving pulse supplied to the electromagnetic actuator 15 and a state of a lens 16a;
Fig. 4 is a diagram illustrating a configuration of an endoscope apparatus configuring a lens driving apparatus according to a second embodiment; and
Fig. 5 is a cross-sectional diagram to explain configurations of an electromagnetic actuator 42 and a zoom lens unit 43.

### Best Mode for Carrying Out the Invention

Some embodiments of the present invention are described below with reference to drawings.

### (First Embodiment)

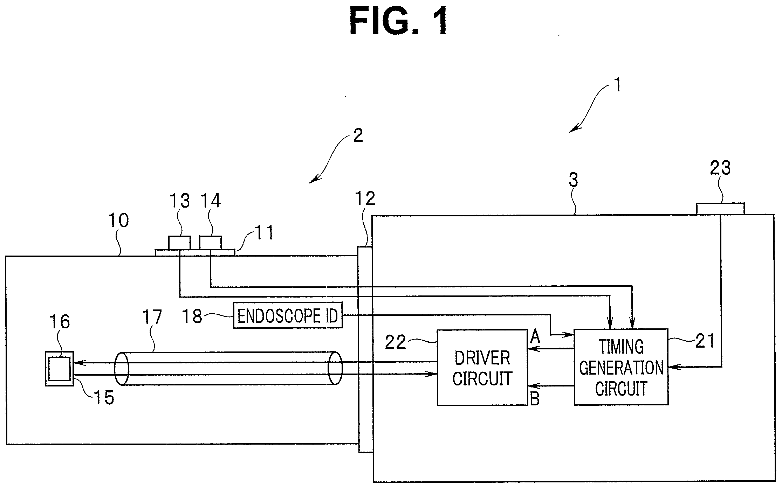

First, a configuration of a lens driving apparatus according to a first embodiment is described with reference to Fig. 1. Fig. 1 is a diagram illustrating a configuration of an endoscope apparatus configuring the lens driving apparatus according to the first embodiment.

As illustrated in Fig. 1, an endoscope apparatus 1 that is the lens driving apparatus includes an endoscope 2 and a driving unit 3 that is provided at a distal end of the endoscope 2 to drive an electromagnetic actuator 15 described later. The endoscope 2 includes: an elongated insertion portion 10 that is to be inserted into a subject; an operation section 11 for various kinds of operation of the endoscope 2, the operation section 11 being provided at a proximal end of the insertion portion 10; and a connector portion 12 provided at a proximal end of the operation section 11. The endoscope 2 is detachably connected to the driving unit 3 through the connector portion 12. The operation section 11 serving as an instruction section includes a Near button 13 to set a focus position at a near point and a Normal button 14 to set the focus position at a normal point.

Further, the endoscope 2 is mounted with the electromagnetic actuator 15 and a focus lens unit 16 that is driven by the electromagnetic actuator 15, at the distal end. A power supply cable 17 is connected to the electromagnetic actuator 15, and a drive current is supplied from the driving unit 3 to the electromagnetic actuator 15.

The driving unit 3 includes: a timing generation circuit 21 that generates a drive pulse; a driver circuit 22 that generates the drive current based on the drive pulse provided from the timing generation circuit 21 and supplies the generated drive current to the electromagnetic actuator 15 through the power supply cable 17; and a setting section 23 to set various kinds of setting of the driving unit 3.

The timing generation circuit 21 serving as a drive signal control section outputs the drive pulse to one of an A input terminal and a B input terminal of the driver circuit 22 according to an operation state of one of the Near button 13 and the Normal button 14 of the operation section 11. Note that the timing generation circuit 21 may be configured of, for example, an FPGA or a microcomputer. Further, the timing generation circuit 21 does not have to be provided in the driving unit 3.

The driver circuit 22, serving as a first drive signal generation section, is a circuit that causes the drive current to flow through the electromagnetic actuator 15 in a predetermined direction and a direction opposite to the predetermined direction, namely, in both directions. The driver circuit 22 is, for example, an H-bridge circuit that is configured of four switching devices (such as MOSFETs, bipolar transistors, or the like). The driver circuit 22 changes the direction of the drive current that is to flow through the electromagnetic actuator 15, based on whether the drive pulse provided from the timing generation circuit 21 is received by the A input terminal or the B input terminal.

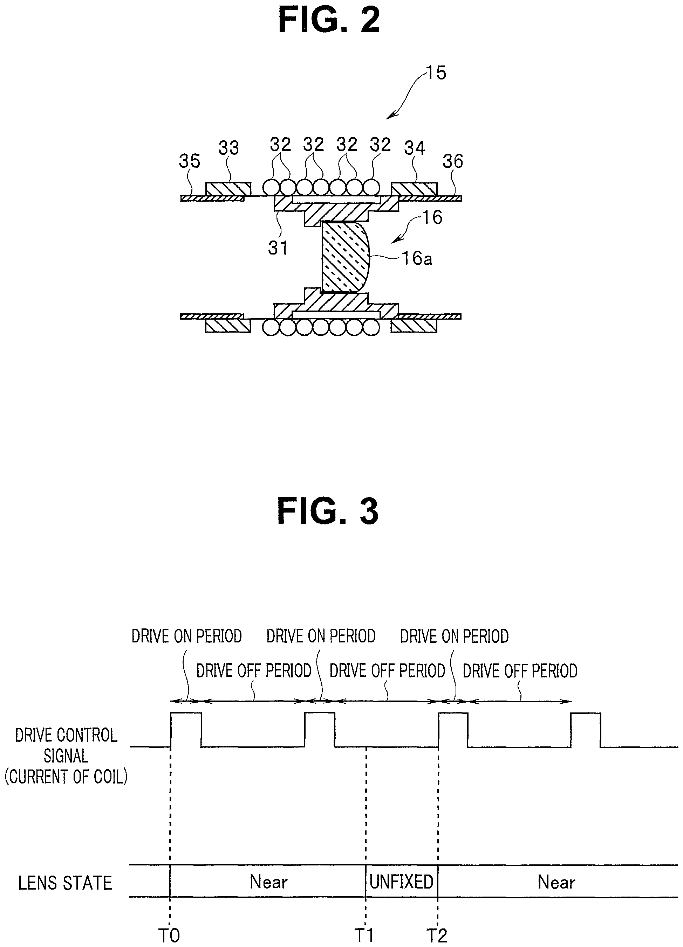

Fig. 2 is a cross-sectional diagram to explain the configurations of the electromagnetic actuator 15 and the focus lens unit 16.

As illustrated in Fig. 2, the electromagnetic actuator 15 includes: a cylindrical soft iron 31; a coil 32 wound around an outer periphery of the soft iron 31; a magnet 33 and a magnet 34 that are respectively provided on one end side (front side of the insertion portion 10) and the other end side (rear side of the insertion portion 10); and a contact part 35 and a contact part 36 with which the soft iron 31 comes into contact.

The cylindrical soft iron 31 configures a lens holding section that so holds a focus lens 16a of the focus lens unit 16 as to be movable between the contact part 35 (one end part) and the contact part 36 (the other end part).

The lens 16a, serving as a first lens, is a focus lens that varies a focal distance of an objective optical system including the lens 16a, along with movement of the soft iron 31. Note that, although the focus lens unit 16 includes one focus lens 16a, the number of the focus lens is not limited to one, and the focus lens unit 16 may include two or more focus lenses. In this case, the soft iron 31, serving as the lens holding section, holds the two or more lenses.

The magnet 33 and the magnet 34 configure a first magnet that attracts and holds the soft iron 31 when the soft iron 31 comes into contact with one of the contact part 35 and the contact part 36.

A method of controlling the electromagnetic actuator 15 is now described.

Fig. 3 is a diagram to explain an example of the drive pulse supplied to the electromagnetic actuator 15 and a state of the lens 16a.

When the Near button 13 for focusing provided in the operation section 11 of the endoscope 2 is pressed, an operation signal of the button is provided to the timing generation circuit 21. When receiving the operation signal that indicates pressing of the Near button 13, the timing generation circuit 21 generates the drive pulse illustrated in Fig. 3, and provides the generated drive pulse to the A input terminal of the driver circuit 22. When receiving the drive pulse through the A input terminal, the driver circuit 22 applies the drive current to the electromagnetic actuator 15 in the predetermined direction through the power supply cable 17.

At this time, the drive current flows through the electromagnetic actuator 15 in the predetermined direction, the soft iron 31 and the lens 16a accordingly move rightward in Fig. 2, and then the right magnet 34 attracts and stops the soft iron 31. In the present embodiment, the pulse is repeatedly and periodically applied to the coil 32 even after the right magnet 34 attracts and stops the soft iron 31, for example, by the pulse at a time T0 in Fig. 3.

As mentioned above, the timing generation circuit 21 generates the drive pulse that causes the soft iron 31 to move in the direction instructed by the operation section 11, and supplies the generated drive pulse to the driver circuit 22. In addition, the timing generation circuit 21 controls the driver circuit 22 to periodically generate the drive signal that causes the soft iron 31 to move in the direction instructed by the operation section 11 even after the soft iron 31 is attracted to the magnet 34.

On the other hand, when the Normal button 14 for focusing provided in the operation section 11 of the endoscope 2 is pressed, an operation signal of the button is provided to the timing generation circuit 21. When receiving the operation signal that indicates pressing of the Normal button 14 for focusing, the timing generation circuit 21 generates the above-described periodic pulse illustrated in Fig. 3, and provides the periodic pulse to the B input terminal of the driver circuit 22. When receiving the drive pulse through the B input terminal, the driver circuit 22 applies the drive current to the electromagnetic actuator 15 in a direction opposite to the predetermined direction, through the power supply cable 17.

At this time, the current flows through the electromagnetic actuator 15 in the direction opposite to the predetermined direction, the soft iron 31 and the lens 16a accordingly move leftward in Fig. 2, and then the left magnet 33 attracts and stops the soft iron 31. In the present embodiment, the pulse is repeatedly and periodically applied to the coil 32 even after the left magnet 33 attracts and stops the soft iron 31, for example, by the pulse at the time T0 in Fig. 3.

Such periodic application of the pulse to the coil 32 draws back the lens 16a toward the contact part 36 by a next pulse, even if any impact is applied to the electromagnetic actuator 15 (or the predetermined position of the endoscope 2) while the soft iron 31 is in contact with the contact part 36, for example.

For example, even if any impact is applied to the electromagnetic actuator 15 of the endoscope 2 at a time T1 in Fig. 3 when the pulse is not applied to the coil 32 and the soft iron 31 is separated from (unfixed to) the contact part 36, the pulse is applied to the coil 32 at a time T2, the soft iron 31 is accordingly drawn back toward the contact part 36, and the magnet 34 attracts (Near) and stops the soft iron 31.

The repetition period of the pulse application is set to a short time (for example, about one second) that does not interfere with diagnosis even if the endoscope observation field is deviated for a moment. Setting the repetition period to a shorter time period suppresses deviation of the endoscope observation field. When the repetition period is set to an excessively-short time period, however, overheat caused by energization of the electromagnetic actuator 15 may occur. Therefore, the repetition period may be desirably set to a time period that does not allow overheat caused by energization of the electromagnetic actuator 15, for example, about one second as mentioned above.

Note that the repetition period of the pulse application is not necessarily constant consistently, and the period may be varied, for example, depending on a pulse to be applied. Further, a user may set the repetition period of the pulse application with use of the setting section 23. Furthermore, the timing generation circuit 21 may vary the repetition period of the pulse application based on the endoscope ID 18 provided from the endoscope 2 that is connected to the driving unit 3.

Moreover, although the focus lens unit 16 is driven with use of the electromagnetic actuator 15 in the present embodiment, the actuator is not limited to the electromagnetic actuator 15, and the focus lens unit 16 may be driven with use of, for example, a piezoelectric actuator or a shape-memory-alloy actuator.

As mentioned above, the endoscope apparatus 1 configuring the lens driving apparatus periodically applies the pulse to the electromagnetic actuator 15 even after the soft iron 31 is attracted to one of the magnet 33 and the magnet 34. As a result, in the endoscope apparatus 1, even if external impact is applied to the electromagnetic actuator 15 (or the predetermined position of the endoscope 2) and the soft iron 31 is accordingly separated from the magnet 33 or 34 during the endoscope observation, the soft iron 31 is immediately attracted to the magnet 33 or 34. Therefore, the endoscope observation is not interfered.

Accordingly, the lens driving apparatus of the present embodiment allows for stable endoscope observation even if any impact is applied to the actuator.

### (Second Embodiment)

Next, a second embodiment is described.

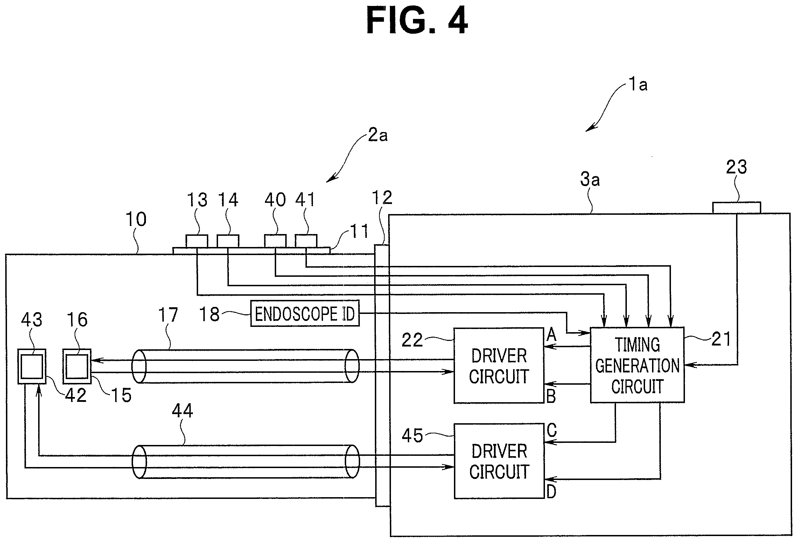

Fig. 4 is a diagram illustrating a configuration of an endoscope apparatus configuring a lens driving apparatus according to a second embodiment. Note that components in Fig. 4 similar to the components in Fig. 1 are denoted by the same reference numerals, and description of such components is omitted.

An endoscope apparatus 1a of the present embodiment is configured to perform zoom adjustment, in addition to the focus adjustment described in the first embodiment. As illustrated in Fig. 4, the endoscope apparatus 1a includes an endoscope 2a and a driving unit 3a.

The endoscope 2a further includes a Tele button 40 and a Wide button 41 for zooming in the operation section 11, as compared with the endoscope 2 of Fig. 1. Moreover, the endoscope 2a further includes, at a distal end, an electromagnetic actuator 42 and a zoom lens unit 43 that is driven by the electromagnetic actuator 42, as compared with the endoscope 2 of Fig. 1. The electromagnetic actuator 42 is connected to a power supply cable 44, and a drive current is supplied from the driving unit 3a to the electromagnetic actuator 42.

The driving unit 3a further includes a driver circuit 45, as compared with the driving unit 3 of Fig. 1. The timing generation circuit 21 provides a drive pulse to one of a C input terminal and a D input terminal of the driver circuit 45 according to an operation state of one of the Tele button 40 and the Wide button 41 of the operation section 11.

The driver circuit 45 is a circuit that causes the drive current to flow through the electromagnetic actuator 42 in a predetermined direction and a direction opposite to the predetermined direction, namely, in both directions, as with the driver circuit 22. Examples of the driver circuit 45 may include an H-bridge circuit. The driver circuit 45 changes the direction of the drive current that is to flow through the electromagnetic actuator 42, based on whether the drive pulse provided from the timing generation circuit 21 is received by the C input terminal or the D input terminal.

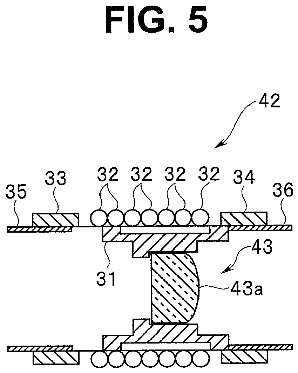

Fig. 5 is a cross-sectional diagram to explain configurations of the electromagnetic actuator 42 and the zoom lens unit 43. Note that components in Fig. 5 similar to the components in Fig. 2 are denoted by the same reference numerals, and description of such components is omitted.

The electromagnetic actuator 42 of the present embodiment has a configuration similar to the configuration of the electromagnetic actuator 15 of the first embodiment, and a zoom lens 43a of the zoom lens unit 43 is held by the soft iron 31. The cylindrical soft iron 31 configures a lens holding section that so holds the zoom lens 43a of the zoom lens unit 43 as to be movable between the contact part 35 (one end part) and the contact part 36 (the other end part).

The lens 43a is a variable power lens that varies a magnification factor of an optical image, along with the movement of the soft iron 31. Note that, although the zoom lens unit 43 includes one zoom lens 43a, the number of the zoom lens is not limited to one, and the zoom lens unit 43 may include two or more lenses.

A method of controlling the electromagnetic actuator 42 is now described. The method of controlling the electromagnetic actuator 42 is substantially similar to the method of controlling the electromagnetic actuator 15. When the Tele button 40 for zooming provided in the operation section 11 of the endoscope 2a is pressed, an operation signal of the button is provided to the timing generation circuit 21. When receiving the operation signal that indicates pressing of the Tele button 40, the timing generation circuit 21 generates the above-described periodic pulse illustrated in Fig. 3, and provides the periodic pulse to the C input terminal of the driver circuit 45. When receiving the drive pulse through the C input terminal, the driver circuit 45 applies the drive current to the electromagnetic actuator 42 in the predetermined direction through the power supply cable 44.

At this time, the current flows through the electromagnetic actuator 42 in the predetermined direction, the soft iron 31 and the lens 43a accordingly move rightward in Fig. 4, and then the right magnet 34 attracts and stops the soft iron 31. In the present embodiment, the right magnet 34 attracts and stops the soft iron 31. Also in the present embodiment, the pulse is repeatedly and periodically applied to the coil 32 even after the right magnet 34 attracts and stops the soft iron 31, for example, by the pulse at a time T0 in Fig. 3.

On the other hand, when the Wide button 41 for zooming provided in the operation section 11 of the endoscope 2a is pressed, an operation signal of the button is provided to the timing generation circuit 21. When receiving the operation signal that indicates pressing of the Normal button 41 for zooming, the timing generation circuit 21 generates the above-described periodic pulse illustrated in Fig. 3, and provides the periodic pulse to the D input terminal of the driver circuit 45. When receiving the drive pulse through the D input terminal, the driver circuit 45 applies the drive current to the electromagnetic actuator 42 in the direction opposite to the predetermined direction, through the power supply cable 44.

At this time, the current flows through the electromagnetic actuator 42 in the direction opposite to the predetermined direction, the soft iron 31 and the lens 43a accordingly move leftward in Fig. 4, and then the left magnet 33 attracts and stops the soft iron 31. Also in the present embodiment, the pulse is repeatedly and periodically applied to the coil 32, even after the left magnet 33 attracts and stops the soft iron 31, for example, by the pulse at the time T0 in Fig. 3.

Such periodic application of the pulse to the coil 32 draws back the lens 43a toward the contact part 36 by a next pulse, for example, even if any impact is applied to the electromagnetic actuator 42 (or the predetermined position of the endoscope 2a) while the soft iron 31 is in contact with the contact part 36. The repetition period of the pulse application is set to a short time period (for example, about one second) that does not interfere with diagnosis even if the endoscope observation field is deviated for a moment, as with the first embodiment.

The repetition period of the pulse application is not necessarily constant consistently, and the period may be varied, for example, depending on the pulse to be applied, as with the first embodiment. Further, the user may set the repetition period of the pulse application with use of the setting section 23. Furthermore, the timing generation circuit 21 may vary the repetition period of the pulse application based on the endoscope ID 18 provided from the endoscope 2a that is connected to the driving unit 3 a.

Moreover, in the present embodiment, the drive pulse to be applied to the electromagnetic actuator 42 is described with reference to Fig. 3, namely, the drive pulse to be applied to the electromagnetic actuator 42 is described as the drive pulse same as the drive pulse to be applied to the electromagnetic actuator 15 in the first embodiment. The drive pulse to be applied to the electromagnetic actuator 42, however, may be different from the drive pulse to be applied to the electromagnetic actuator 15.

Further, the endoscope apparatus 1a has the configuration to perform the focus adjustment and the zoom adjustment, however, the electromagnetic actuator 15, the focus lens unit 16, the power supply cable 17, and the driver circuit 22 may be removed from the endoscope apparatus 1 a, and the endoscope apparatus 1a may have a configuration to perform only the zoom adjustment.

As mentioned above, the endoscope apparatus 1 a configuring the lens driving apparatus periodically applies the pulse to the electromagnetic actuator 42 even after the soft iron 31 is attracted to the magnet 33 or 34. As a result, in the endoscope apparatus 1, even if external impact is applied to the electromagnetic actuator 42 (or the predetermined position of the endoscope 2a) and the soft iron 31 is accordingly separated from the magnet 33 or 34 during the endoscope observation, the soft iron 31 is immediately attracted to the magnet 33 or 34. Therefore, the endoscope observation is not interfered.

Accordingly, the lens driving apparatus of the present embodiment allows for stable endoscope observation even if any impact is applied to the actuator, as with the first embodiment.

The present invention is not limited to the above-described embodiments, and various modifications, alternations, and the like may be performed without departing from the scope of the present invention.

The present application is based upon and claims the benefit of priority of the Japanese Patent Application No. 2014-146196 filed in the Japan Patent Office on July 16, 2014, the disclosed contents of which are incorporated in the specification, the claims, and the drawings of the present application by reference.

## Claims

1. A lens driving apparatus, comprising:
a first lens;
a first lens holding section that holds the first lens to be movable between an end part and another end part;
a first magnet that is provided on the end part and the other end part, and attracts and holds the first lens holding section when the first lens holding section comes into contact with one of the end part and the other end part;
a first drive signal generation section that generates a drive signal, the drive signal bringing the first lens holding section into contact with one of the end part and the other end part;
an instruction section that instructs the first drive signal generation section to move the first lens holding section to bring the first lens holding section into contact with the end part or move the first lens holding section to bring the first lens holding section into contact with the other end part; and
a drive signal control section that generates a drive signal to supply the drive signal to the first drive signal generation section, the drive signal causing the first lens holding section to move in a direction instructed by the instruction section, and controls the first drive signal generation section to periodically generate the drive signal even after the first lens holding section comes into contact with the instructed end part, the drive signal causing the first lens holding section to move toward the instructed end part.

2. The lens driving apparatus according to claim 1, wherein the first lens is a focus lens that varies a focal distance of an objective optical system including the lens, along with the movement.

3. The lens driving apparatus according to claim 1, wherein the first lens is a variable power lens that varies a magnification factor of an optical image, along with the movement.

4. The lens driving apparatus according to claim 1, wherein
the first lens holding section includes an outer periphery wound with a coil, and
the first drive signal generation section applies a current to the coil in a predetermined direction or a direction opposite to the predetermined direction, to bring the first lens holding section into contact with one of the end part and the other end part.

5. The lens driving apparatus according to claim 1, further comprising:
a second lens;
a second lens holding section that holds the second lens to be movable between the end part and the other end part;
a second magnet that is provided on the end part and the other end part, and attracts and holds the second lens holding section when the second lens holding section comes into contact with one of the end part and the other end part; and
a second drive signal generation section that generates a drive signal, the drive signal bringing the second lens holding section into contact with one of the end part and the other end part.

6. The lens driving apparatus according to claim 5, wherein
the first lens is a focus lens that varies a focal distance of an objective optical system including the lens, along with the movement, and
the second lens is a variable power lens that varies a magnification factor of an optical image, along with the movement.

7. The lens driving apparatus according to claim 1, further comprising a setting section that optionally sets a period in which the drive signal is periodically generated.
